(19) Europäisches Patentamt
European Patent Office
Office européen des brevets

(11) **EP 2 877 130 B1**

(12) **FASCICULE DE BREVET EUROPEEN**

(45) Date de publication et mention de la délivrance du brevet:
**22.02.2017 Bulletin 2017/08**

(21) Numéro de dépôt: **13742445.3**

(22) Date de dépôt: **26.07.2013**

(51) Int Cl.:
*A61F 2/64* (2006.01)     *A61F 2/66* (2006.01)
*A61F 2/68* (2006.01)     *A61F 2/70* (2006.01)
*A61F 2/74* (2006.01)     *A61F 2/76* (2006.01)

(86) Numéro de dépôt international:
**PCT/EP2013/065845**

(87) Numéro de publication internationale:
**WO 2014/016424 (30.01.2014 Gazette 2014/05)**

(54) **SYSTÈME HYDRAULIQUE D'ENSEMBLE GENOU-CHEVILLE CONTROLÉ PAR MICROPROCESSEUR**

HYDRAULIKSYSTEM FÜR EINE MIKROPROZESSORGESTEUERTE KNIE-FUSSGELENK-ANORDNUNG

HYDRAULIC SYSTEM FOR A KNEE-ANKLE ASSEMBLY CONTROLLED BY A MICROPROCESSOR

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **27.07.2012 EP 12305924**

(43) Date de publication de la demande:
**03.06.2015 Bulletin 2015/23**

(73) Titulaire: **Proteor**
**21850 Saint-Apollinaire (FR)**

(72) Inventeurs:
• **BONNET, Xavier**
  **21000 Dijon (FR)**
• **DJIAN, Francis**
  **21110 Genlis (FR)**

(74) Mandataire: **Cabinet Laurent & Charras**
**3 place de l'Hotel de Ville**
**CS 70203**
**42005 Saint-Etienne Cedex 1 (FR)**

(56) Documents cités:
**US-A- 2 478 721     US-A1- 2009 299 489**
**US-A1- 2010 241 242     US-A1- 2011 098 828**
**US-A1- 2011 166 489**

**EP 2 877 130 B1**

**Description**

**DOMAINE TECHNIQUE**

La présente invention concerne une prothèse de genou-cheville destinée à des personnes amputées du membre inférieur et plus particulièrement un système hydraulique pour l'articulation du genou et de la cheville contrôlé par un microprocesseur.

**ART ANTERIEUR**

Il est bien connu que les prothèses pour amputé fémoral sont composées généralement d'un genou prothétique et d'un ensemble pied cheville. Il existe une multitude de genoux prothétiques ; toutefois, on peut distinguer deux types de genoux prothétiques : les genoux passifs et les genoux actifs.

Les genoux mécatroniques passifs, contrôlés par microprocesseur, sont dits passifs dans le sens où ils n'apportent pas d'énergie mais gèrent l'énergie issue de la marche. Ils comprennent un vérin hydraulique ou magnéto-rhéologique qui contrôle le mouvement de flexion et d'extension du genou, par l'intermédiaire d'une ou plusieurs électrovannes dont le débit est piloté par un microprocesseur. Le freinage engendré par le vérin est fort en phase d'appui, afin d'éviter la chute, et faible en phase oscillante pour gérer le mouvement pendulaire de la jambe.

Les genoux actifs, quant à eux, comprennent un moteur qui apporte une énergie supplémentaire à celle issue de la marche.

Tous ces genoux comprennent plusieurs capteurs permettant de déterminer quand modifier les positions des électrovannes ou d'adapter le couple moteur. Des capteurs permettent de reconnaitre l'instant du cycle de marche dans lequel on se trouve tel que la phase d'appui ou la phase oscillante par exemple et ils permettent également de mesurer la vitesse de marche.

Les dispositifs les plus récents portent sur la reconnaissance de la situation dans laquelle l'amputé se trouve (Pente, escaliers, position assise, etc.) tels que le Genium® (genou passif commercialisé par la société Otto Bock) et le POWER KNEE® (genou actif commercialisé par la société Össur-Victhom) décrit notamment dans les demandes de brevet américain US 2004/0111163 et US 2006/0122711. La reconnaissance des situations nécessite l'utilisation d'un plus grand nombre de capteurs et d'algorithmes de reconnaissances plus élaborés. La reconnaissance de ces situations permet d'adapter le réglage des résistances à la flexion et à l'extension à la situation rencontrée par la personne appareillée.

On connait notamment le C-Leg®, commercialisé par la société Otto Bock, qui est historiquement le premier genou passif à vérin hydraulique piloté par microprocesseur en phase d'appui et en phase oscillante. Ce genou est notamment décrit dans la demande de brevet international WO 96/41599 et dans la demande de brevet européen EP549855. Il contrôle la flexion et l'extension tout au long du cycle de marche (en phase d'appui et en phase oscillante). Plus récemment, la société Otto Bock a introduit le genou Genium® qui est une évolution notable du C-Leg. Ce genou reste un genou passif mais intégrant de nombreuses améliorations liées à la reconnaissance de situations complexes tels que la montée d'escaliers, les pentes, la position assise, etc...

On connait également le genou RHEO KNEE®, commercialisé par la société Össur, qui est un genou passif à vérin magnéto-rhéologique. Ce genou est notamment décrit dans les demandes de brevet international WO 2005/0283257 et WO 2005/087144.

Les études d'analyse des mouvements ont montré que lors de la descente d'escaliers, le couple de freinage du genou prothétique C-Leg® restait plus faible que le couple de freinage du membre controlatéral. Ceci entraine une « chute » sur le membre controlatéral et une augmentation notable de l'effort repris par celui-ci. Cet effort entraine une augmentation des couples articulaires dans l'ensemble du membre controlatéral. De plus pour pouvoir descendre un escalier avec un genou « conventionnel », il faut placer le pied prothétique sur le nez de la marche afin de permettre le déroulé du pied et l'avancé du tibia. Ce placement doit être précis ce qui demande une attention particulière de l'utilisateur. Les personnes appareillées évitent donc de descendre les escaliers en pas alternés dès que l'escalier est trop raide, que le sol est glissant, etc...

On connait également des prothèses pied cheville qui peuvent être classés suivant trois catégories : les chevilles purement passives, les chevilles mécatroniques passives avec électrovannes et les chevilles mécatroniques actives. Les chevilles purement passives ne comportent ni capteurs ni électrovannes ni motorisation. Les chevilles passives avec électrovannes permettent de piloter les amortissements hydrauliques et les chevilles mécatroniques incluent des capteurs, des électrovannes ou un moteur.

Le pied cheville PROPRIO FOOT®, commercialisé par la société Össur et divulgué notamment dans les demandes de brevet international WO 2005/0197717 et WO 20070050047, est une prothèse mécatronique dont l'articulation de cheville comprend un moteur électrique piloté par des capteurs. Une telle disposition permet de relever le bout du pied pendant la phase oscillante pour éviter que celui-ci ne heurte le sol, de modifier le réglage de hauteur de talon (si l'utilisateur change de chaussures), d'adapter l'angle de la cheville en phase d'appui en fonction de la pente et en escaliers, cette adaptation étant toujours faite lorsque la cheville est déchargée du poids de l'utilisateur, en phase pendulaire, et de modifier l'angle de la cheville en position assise et lors du transfert position assise/ position debout.

On notera que, bien que le moteur ne serve qu'en phase oscillante, il est cependant déjà volumineux, fait du bruit, et consomme beaucoup d'énergie puisque c'est le moteur qui permet d'entrainer le mouvement du pied en phase oscillante à chaque pas. Le ProprioFoot® impose à l'utilisateur de porter une batterie volumineuse et de la recharger tous les jours. De plus le moteur est susceptible de faire du bruit.

On connait également le pied cheville Echelon® commercialisé par la société Endolite et décrit notamment dans la demande de brevet international WO2008103917, qui est une cheville purement passive intégrant un petit vérin hydraulique linéaire permettant une mobilité de 3° en dorsiflexion (sens de rotation du pied qui amène les orteils vers le haut) et de 6° en plantiflexion (rotation qui amène les orteils vers le bas). Deux vannes à pointeaux réglables manuellement permettent d'ajuster le débit séparément en dorsiflexion et en plantiflexion. La plantiflexion est notamment intéressante pour les descentes de pente ce qui permet de mettre le pied rapidement à plat.

Toutefois, la mobilité en dorsiflexion est limitée (3°). En effet, le compromis choisi permet de satisfaire à la marche à plat et à la montée de pente.

Par ailleurs, le pied cheville Elan® est une cheville mécatronique passive, évolution du pied cheville Echelon® dans laquelle les vannes manuelles de dorsiflexion et plantiflexion ont été remplacées par des électrovannes. Ceci permet d'ajuster l'amortissement hydraulique en fonction de pente et la vitesse de marche, les amplitudes de mobilité en dorsiflexion et plantiflexion n'ayant pas été modifiées. Cette solution permet de ne modifier les résistances hydrauliques que lorsque le changement de pente est détecté, c'est-à-dire peu fréquemment, ce qui est peu consommateur en batterie. Il est possible d'augmenter l'amplitude de dorsiflexion en montée et l'amplitude de plantiflexion en descente. Cependant l'encombrement semble limiter le développement d'une telle solution.

On connait également le pied cheville Motion Foot®, commercialisé par la société Fillauer, qui est une cheville purement passive avec un fonctionnement similaire à l'Echelon®, à la différence que le cylindre hydraulique est rotatif et non pas linéaire, ainsi que le pied cheville Raize®, commercialisé par la société Fillauer et décrit dans le brevet américain US 6,443,993 notamment, qui est une cheville mécatronique passive intégrant un vérin linéaire hydraulique pilotée par une vanne contrôlée par microprocesseur et des capteurs. La vanne est sans cesse ajustée et permet de contrôler la résistance et l'amplitude de dorsiflexion ainsi que la résistance en plantiflexion. Les fonctions réalisées sont donc plus importantes que l'Elan®, commercialisé par la société Endolite, mais la vanne est sans cesse pilotée ce qui est très consommateur en énergie.

De la même manière que l'Echelon® commercialisé par la société Endolite, le Raize™ ne bouge pas en phase oscillante.

Toutes ces chevilles (Echelon®, Elan®, Raize™) ne permettent donc pas d'augmenter la distance orteil sol au cours de la phase oscillante. La personne appareillée va donc augmenter cette distance en élevant son bassin du côté de la prothèse au cours de la phase oscillante. Cette stratégie est couteuse en énergie (élévation du centre de masse) et entraine des torsions dans le bassin et dans le bas du dos.

Enfin, on connaît des ensembles genou-cheville tels que l'Hydracadence, décrit notamment dans le brevet américain US 2,478,721, qui est un ensemble genou cheville selon le préambule de la revendication 1 annexée, comportant un vérin hydraulique gérant l'angle du genou et un autre vérin hydraulique gérant l'angle de la cheville. Il y a une liaison hydraulique entre ces 2 cylindres qui permet une synergie entre l'angle de la cheville et l'angle du genou. Les fonctions fournies par ce genou sont une flexion de la cheville pendant la phase pendulaire qui permet d'éviter de heurter le bout du pied au sol, cette dorsiflexion en phase oscillante étant entrainée par la flexion du genou, un réglage de la butée de dorsiflexion qui permet d'adapter la prothèse au changement de la chaussure (hauteur du talon), et une plantiflexion libre qui facilite l'attaque talon.

Cet ensemble genou-cheville ne comprend qu'une seule vanne permettant de régler l'amortissement du genou en phase pendulaire. Par ailleurs, cette prothèse dont la conception remonte aux années 1940 ne comprend pas d'aspect mécatronique donc pas d'adaptation aux différentes situations de marche.

## EXPOSE DE L'INVENTION

L'un des buts de l'invention est donc de remédier à ces inconvénients en proposant une prothèse genou-cheville de conception simple et peu onéreuse et permettant de reproduire les mouvements procurés par des muscles bi-articulaires tels que le muscle gastrocnémien.

A cet effet et conformément à l'invention, il est proposé une prothèse fémorale genou-cheville selon la revendication 1 annexée.

Les déplacements des vérins sont régulés par une ou plusieurs vanne(s).

La ou lesdites vanne(s) sont contrôlées par l'unité de commande en fonction de la phase du cycle de marche et de la situation dans laquelle la personne se trouve.

Ladite phase de marche et/ou la situation sont déterminées par l'unité de commande à partir de données issues de capteurs placés sur l'articulation de genou et/ou l'articulation de cheville et/ou les segments adjacents à ces articulations.

Par ailleurs, chaque capteur consiste en un capteur cinétique, cinématique ou inertiel.

De plus, l'unité de commande pilote la vanne de la conduite et/ou les vannes de régulations des vérins de manière à faire varier les résistances à la flexion/extension de l'articulation de genou et/ou de l'articulation de cheville.

La flexion du genou autorise une dorsiflexion de cheville proportionnelle au mouvement du genou au cours de la phase d'appui et en ce que la flexion du genou entraine une dorsiflexion de la cheville au cours de la phase oscillante.

Le rapport entre la flexion du genou et la dorsiflexion de cheville est compris entre 1/4 et 2/5 et le rapport entre la flexion du genou et la dorsiflexion de cheville est sensiblement égal à 1/3.

De préférence, l'unité de commande comporte au moins un microprocesseur.

Par ailleurs, l'unité de commande comporte un algorithme déterminant le moment de l'articulation de genou ou de cheville en fonction d'un angle d'un segment adjacent à l'articulation par rapport à un référentiel et/ou de la vitesse angulaire du segment adjacent à l'articulation dans un référentiel déterminé et un algorithme déterminant une instruction de pilotage des vérins en fonction du rapport entre le moment de force de l'articulation et un angle d'un segment adjacent à l'articulation par rapport à un référentiel.

Ce contrôle bi-articulaire (genou-cheville) est primordial pour tous les mouvements dits de triple flexion (flexion/extension de la hanche, du genou et de la cheville) tels que la transition entre la position debout et assise, la descente d'escalier avec le pied à plat sur la marche, les mouvements de squat, etc. L'invention permet à une personne amputée du membre inférieur de réaliser notamment ces mouvements de triple flexion et de les contrôler à partir de leur membre résiduel (hanche).

Ce contrôle bi-articulaire est adapté par le microprocesseur en fonction de la phase du cycle de marche (appui/oscillante) et en fonction de la situation dans laquelle la personne se situe (escalier, pente,...).

## BREVE DESCRIPTION DES FIGURES

D'autres avantages et caractéristiques ressortiront mieux de la description qui va suivre, de plusieurs variantes d'exécution, données à titre d'exemple non limitatifs, de la prothèse fémorale genou-cheville conforme à l'invention, en référence aux dessins annexés sur lesquels :

- la figure 1 est une représentation de la prothèse fémorale genou-cheville conforme à l'invention,
- la figure 2 est une représentation schématique du système hydraulique de la prothèse conforme à l'invention,
- la figure 3 est une représentation graphique de l'évolution des angles du genou et de la cheville pendant le cycle de marche à plat, le cycle de marche correspondant à l'intervalle entre 2 appuis talon du même pied,
- la figure 4 est une représentation graphique de l'évolution des angles du genou et de la cheville pendant un cycle de marche dans lequel la marche à plat intègre une flexion du genou en phase d'appui dit « stanceflex »,
- les figure 5A à 5F sont des représentations schématiques du système hydraulique de la prothèse conforme à l'invention, respectivement lors de l'attaque du talon et la mise à plat du pied sur le sol (A), lors du retour du tibia à la verticale (B), lors de la propulsion (C), lors de l'initiation de la flexion du genou (D), lors de la flexion du genou en phase oscillante (E) et lors du retour en extension du genou (F),
- la figure 6 est une représentation schématique d'une variante d'exécution du système hydraulique de la prothèse conforme à l'invention,
- les figure 7A à 7F sont des représentations schématiques de la variante d'exécution du système hydraulique de la prothèse conforme à l'invention, respectivement lors de l'attaque du talon et la mise à plat du pied sur le sol (A), lors du retour du tibia à la verticale (B), lors de la propulsion (C), lors de l'initiation de la flexion du genou (D), lors de la flexion du genou en phase oscillante (E) et lors du retour en extension du genou (F),
- la figure 8 est une représentation graphique de l'évolution des angles du genou et de la cheville lors de la descente d'escalier,
- la figure 9 est une représentation graphique du couplage entre les angles du genou et de la cheville de la prothèse fémorale genou-cheville conforme à l'invention, en phase d'appui,
- la figure 10 est une représentation schématique du passage de la position debout à la position assise (a à d) et de la position assise à la position debout (d à g),
- la figure 11 est une représentation graphique en fonction du pourcentage de cycle de marche (%) de l'angle (exprimés en °), du couple (exprimé en N.m/kg) et de la puissance (exprimé en W/kg) de la cheville, du genou et de la hanche pour des sujets sains pour la marche à plat (en trait plein), pour la descente d'une pente à 12% (en pointillé) et pour la montée d'une pente à 12% (en tirets),
- la figure 12 est une représentation schématique sous forme de diagramme des états et transitions de l'organe de commande en fonction des variables mesurées et estimées,

- la figure 13 est une représentation graphique sans échelle du couple de la cheville en fonction de l'angle du tibia par rapport à la verticale lors de la marche sur le plat, en montée et en descente de pente entre 30% et 50% du cycle de marche.

## DESCRIPTION DETAILLEE DE L'INVENTION

Par souci de clarté, dans la suite de la description, les mêmes éléments ont été désignés par les mêmes références aux différentes figures. De plus, les diverses vues en coupe ne sont pas tracées à l'échelle.

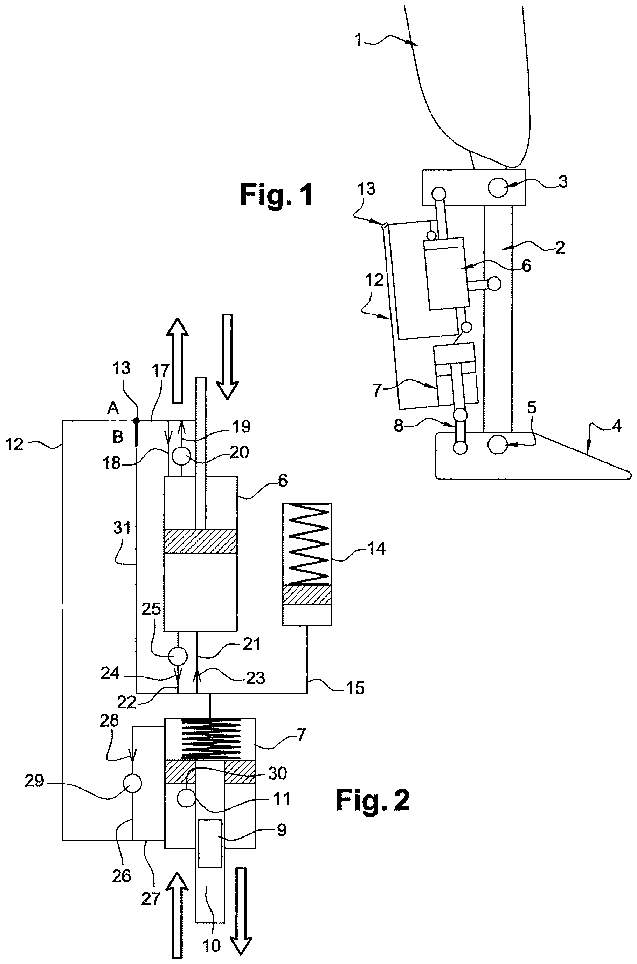

En référence à la figure 1, la prothèse fémorale genou-cheville suivant l'invention comporte un segment fémoral (1) apte à être fixé à une connexion fémorale, et un segment tibial (2) articulé au segment fémoral (1) autour d'une articulation (3) reproduisant les mouvements du genou, ledit segment tibial (2) étant articulé à un pied (4) autour d'une articulation (5) reproduisant les mouvements de la cheville. Par ailleurs, la prothèse comporte un premier vérin hydraulique (6) double effet dont les extrémités sont respectivement solidaires du segment fémoral (1) et du segment tibial (2), et un second vérin hydraulique (7) double effet dont les extrémités sont respectivement solidaires du segment tibial (2) et du pied (4) par l'intermédiaire d'une bielle (8).

En référence aux figures 1 et 2, le second vérin hydraulique (7) comporte un tiroir (9) monté à coulissement dans la tige creuse (10) du second vérin (7), ladite tige creuse (10) comportant au moins un orifice (11) permettant de mettre en communication les deux chambres du vérin (7), en fonction de la position dudit tiroir (9) dans la tige creuse (10). La chambre supérieure du premier vérin hydraulique (6) est connectée à la chambre inférieure du second vérin hydraulique (7) au moyen d'une conduite (12) munie d'une vanne tout ou rien (13) pilotée par un microprocesseur, non représenté sur les figures, en fonction de la phase de la marche telle que la phase d'appui ou la phase oscillante et/ou de la situation telle que la descente d'un escalier, une pente ou une station debout ou analogue comme il sera détaillé plus loin. On notera que le microprocesseur pilote également le tiroir (9) en fonction de la phase de la marche telle que la phase d'appui ou la phase oscillante et/ou de la situation telle que la descente d'un escalier, une pente ou une station debout ou analogue.

Il est bien évident que le microprocesseur pourra être substitué par toute unité de commande équivalente bien connue de l'homme du métier telle qu'un microcontrôleur par exemple sans pour autant sortir du cadre de l'invention.

Par ailleurs, les chambres du premier et du second vérin (6,7) sont connectées à un accumulateur hydraulique (14) par une conduite (15) également en communication avec l'une des voies de la vanne tout ou rien (13). Une seconde voie de la vanne tout ou rien (13) est connectée à deux conduites (16) et (17) communiquant avec la première chambre du premier vérin hydraulique (6). La première conduite (16) comporte un clapet anti-retour (18) et la seconde conduite (17) comporte un clapet anti-retour (19) et une électrovanne (20) contrôlant l'extension du genou. La seconde chambre du premier vérin (6) est connectée à la conduite (15) par deux conduites (21) et (22), la première conduite (21) comportant un clapet anti-retour (23) et la seconde conduite (22) comportant un clapet anti-retour (24) et une électrovanne (25) contrôlant la flexion du genou. La conduite (12) est connectée aux deux chambres du second vérin hydraulique (7) par deux branches (26) et (27), la branche (26) comportant un clapet anti-retour (28) et une électrovanne (29) contrôlant la plantiflexion de la cheville. De plus, la tige creuse (10) du second vérin (7) comporte un orifice(s) (11) permettant de mettre en communication les deux chambres du vérin (7), en fonction de la position dudit tiroir (9) dans la tige creuse (10), et muni d'une électrovanne (30) contrôlant la dorsiflexion de la cheville. La conduite (15) est en communication avec l'une des voies de la vanne tout ou rien (13) via une conduite (31).

Il est bien évident que la tige creuse (10) du second vérin (7) pourra comporter une pluralité d'orifices (11) sans pour autant sortir du cadre de l'invention. Par ailleurs, le nombre et le diamètre des orifices (11) pourront être aisément déterminés par l'Homme du métier.

Lorsque la vanne tout ou rien (13) est en position dite A, lors de la flexion, l'huile arrivant dans la chambre supérieure du premier vérin (6) est obligée de passer par la conduite (12) via la seconde conduite (18) depuis la chambre inférieure du deuxième vérin (7). En phase oscillante, cela provoque une dorsiflexion de la cheville. En phase d'appui, le mouvement de dorsiflexion est autorisé, mais est réalisé en fonction du bilan des pressions s'exerçant sur le piston du deuxième vérin (7), l'huile pouvant transiter via les régulations en plantiflexion et en dorsiflexion, c'est-à-dire l'électrovanne (29) et respectivement l'électrovanne (30).

Lors de l'extension du genou, l'huile sortant de la chambre supérieure du premier vérin (6) transite par la conduite (12) via le clapet anti-retour (19) jusqu'à la chambre inférieure du deuxième vérin (7). En phase oscillante, cela entraine automatiquement un retour en plantiflexion de la cheville. En phase d'appui le mouvement est autorisé ou imposé en fonction de la position du piston du deuxième vérin (7) par rapport au tiroir (9).

Lorsque la vanne tout ou rien (13) est en position dite B, l'huile sortant et arrivant dans la chambre supérieure du premier vérin (6) transite directement vers l'accumulateur (14) via la conduite (15) sans passer par le deuxième vérin (7). Ceci permet un fonctionnement découplé des deux vérins (6) et (7).

Les électrovannes (20,25,29,30) pourront être substituées par toute autre vanne bien connue de l'homme du métier afin de réguler les déplacements des vérins (6,7) sans pour autant sortir du cadre de l'invention.

Lesdites vannes proportionnelles (20,25,29,30) sont contrôlées par le microprocesseur en fonction de la phase du cycle de marche et de la situation dans laquelle la personne se trouve. La phase de marche et/ou la situation sont déterminées par le microprocesseur à partir de données issues de capteurs, non représentés sur les figures, placés sur l'articulation de genou et/ou l'articulation de cheville et/ou le tibia et/ou les vérins. Chaque capteur consiste en un capteur cinétique, cinématique ou inertiel ou tout autre capteur équivalent bien connu de l'homme du métier tel qu'un gyroscope ou un accéléromètre par exemple. Ledit microprocesseur pilote la vanne tout ou rien (13) de la conduite (12) et les vannes proportionnelles (20,25,29,30) de régulations des vérins (6,7) de manière à faire varier les résistances à la flexion/extension de l'articulation de genou et/ou de l'articulation de cheville. La flexion du genou autorise une dorsiflexion de cheville proportionnelle au mouvement du genou au cours de la phase d'appui et la flexion du genou entraine une dorsiflexion de la cheville au cours de la phase oscillante.

On observera que la prothèse fémorale suivant l'invention comporte ainsi un premier vérin (6) dit de genou et un second vérin (7) dit de cheville dont les corps sont mécaniquement solidaires et couplés hydrauliquement. Ce couplage, qu'on nommera « synergie genou cheville », consiste en un partage d'énergie hydraulique entre les deux vérins (6,7) de sorte que, selon l'état de la synergie, la flexion du genou est capable de provoquer (phase pendulaire) ou autoriser (phase appui) une flexion de la cheville ou inversement. Cette synergie peut être active ou inactive selon les situations de marche. La prothèse suivant l'invention procure un gain de poids et d'encombrement grâce à la mutualisation des organes mécaniques, permet de placer des capteurs sur le genou et la cheville afin de faciliter la reconnaissance des situations de marche en combinant plusieurs capteurs, permet de partager la batterie, et permet de contrôler la position de la cheville en fonction du genou et vice versa.

La prothèse suivant l'invention permet notamment de relever le pied automatiquement en phase oscillante sans avoir besoin de moteur, uniquement grâce à la flexion du genou, de plier automatiquement la cheville lorsqu'on fléchit les genoux par exemple dans un mouvement de passage debout vers assis, en liant l'angle de flexion du genou et l'angle de dorsiflexion de la cheville.

On observera que cette synergie existe naturellement dans le corps humain par le biais des muscles bi-articulaires tels que le gastrocnémien.

Pour le transfert debout-assis, la descente des escaliers, les mouvements de squat, le ratio entre la flexion du genou et la dorsiflexion de la cheville est comprise entre 1/4 et 2/5, et de préférence de l'ordre de 1/3.

Les mouvements peuvent être combinés (en synergie) ou dissociés. La synergie peut être activée ou désactivée par l'actionnement de la vanne tout ou rien (12) pilotée par un organe de commande à microprocesseur.

Il est bien évident que la synergie pourra être constamment active sans pour autant sortir du cadre de l'invention.

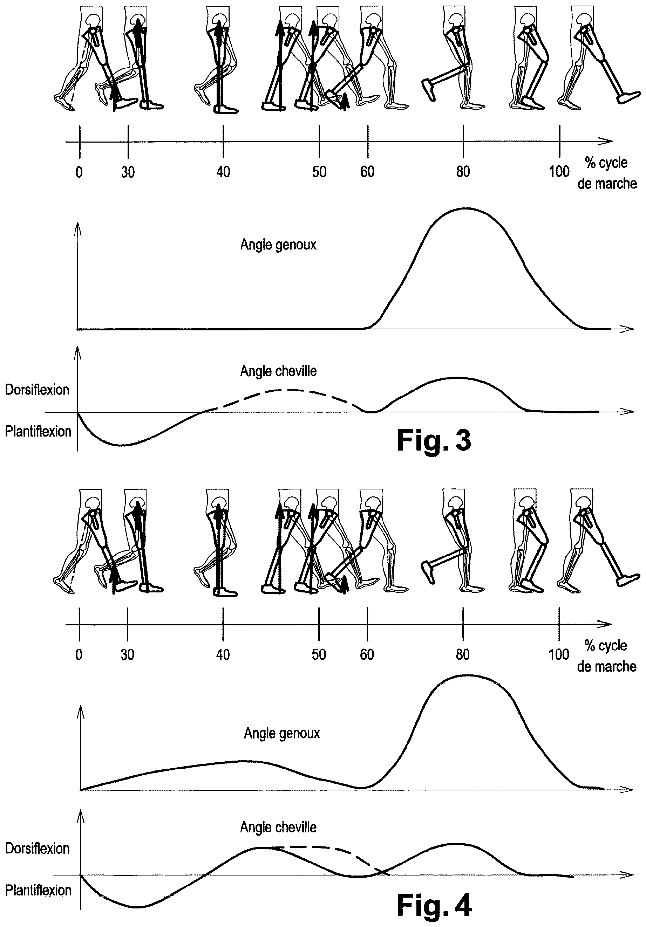

En référence à la figure 3, la marche à plat peut être décomposée en plusieurs parties. De 0 à 12%, le pied se met à plat rapidement sur le sol par une plantiflexion de la cheville. La flexion du genou est contrôlée par une résistance à la flexion importante du genou. De 12 à 30%, le tibia repasse à la verticale, la cheville retourne en position neutre en position de butée de dorsiflexion. De 30 à 48%, le genou est en extension complète, et la cheville en position neutre. La progression du tibia est rendu possible par la déformation de la lame de pied entrainant une dorsiflexion apparente représentée par le trait pointillé sur la figure. De 48 à 60%, la résistance à la flexion du genou est abaissée pour permettre l'initiation de la phase oscillante. Le genou fléchit rapidement pour atteindre 30° à la fin de la phase d'appui. La lame de pied reprend sa forme non déformée. La dorsiflexion de la cheville dépend du mode de synergie (voir plus loin). De 60 à 80%, le genou poursuit sa flexion en phase oscillante. La dorsiflexion de la cheville est proportionnelle à la flexion du genou augmentant la distance entre les orteils et le sol afin d'éviter le risque de trébuchage. De 80 à 100%, le genou revient en extension, la résistance de contrôle de la flexion est augmentée pour freiner le genou en cas de trébuchage et pour préparer la phase d'appui suivante. Le comportement de la cheville dépend du mode de synergie genou cheville.

En référence à la figure 4, la marche à plat intégrant une flexion du genou en phase d'appui, ladite flexion étant communément appelée « stanceflex » peut être décomposée en plusieurs parties. De 0 à 12%, le pied se met à plat rapidement sur le sol par une plantiflexion de la cheville. En modifiant la résistance à la plantiflexion et en ajustant la résistance à la flexion du genou, la mise à plat du pied peut se faire par une combinaison de la flexion du genou et de la plantiflexion de cheville. De 12 à 30%, la flexion du genou autorise une dorsiflexion proportionnelle de la cheville (synergie). Le tibia passe donc la verticale en conservant une flexion du genou. De 30 à 48%, le genou repasse en extension complète entrainant un retour en position neutre de la cheville. L'énergie nécessaire pour freiner le retour en extension du genou est donc transmise à la cheville permettant une propulsion du centre de gravité vers le haut et vers l'avant. Ceci à pour intérêt de préparer la transition du poids sur le membre controlatéral.

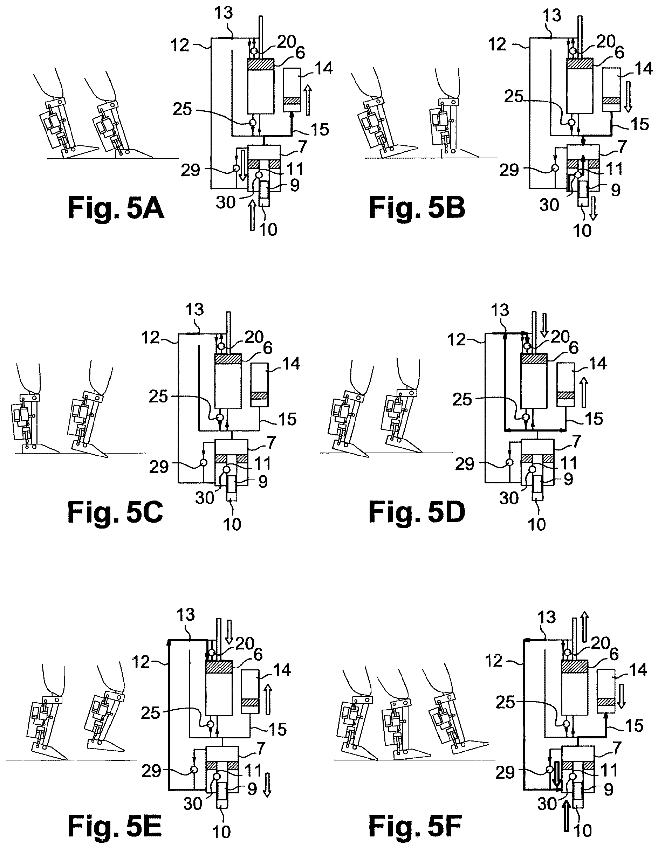

On expliquera maintenant le fonctionnement de la prothèse suivant l'invention en référence aux figures 5A à 5F.

En référence à la figure 5A, lors de la plantiflexion, c'est-à-dire de de 0 à 12% du cycle de marche représenté sur la figure 3, l'appui sur le talon provoque un déplacement du piston de cheville vers le haut. L'huile de la chambre supérieure du vérin (7) de cheville passe dans la chambre inférieure via l'électrovanne (29) de régulation de plantiflexion. L'excédent d'huile est envoyé vers l'accumulateur (14). Afin de réguler la plantiflexion, soit une vanne manuelle ajustée par l'orthoprothésiste en fonction du poids et du style de marche du patient est utilisée soit une électrovanne est utilisée pour permettre aussi l'adaptation à des situations nécessitant un amortissement plus ou moins fort. Cette phase se termine lorsque le pied est à plat sur le sol ou que le piston de cheville est en position extrême haute.

Si la vanne tout ou rien dite de synergie (13) est en position B, le genou ne fléchit pas et il n'y pas de « stanceflex ». Par contre, si la vanne tout ou rien dite de synergie (13) est en position A, l'huile peut passer de la chambre inférieure du vérin (7) pied à la chambre supérieure du vérin (6) du genou entrainant la flexion de genou en phase d'appui (stanceflex). Cette flexion est contrôlée par la régulation de la flexion du genou (résistance importante), c'est-à-dire par l'électrovanne (25).

On notera que le réglage des électrovannes (29) et (25) permet de choisir plus ou moins de plantiflexion ou de « stanceflex » en début d'appui en fonction des préférences de marche de l'utilisateur. Si ces vannes (29) et (25) sont pilotées par le microprocesseur, la plantiflexion et le stanceflex sont ajustés en fonction de la situation (pente, vitesse, port de charge, etc...).

En référence à la figure 5B, lors du retour du tibia à la verticale, c'est-à-dire de 12 à 30% du cycle de marche représenté sur la figure 3, une fois le pied à plat, le tibia repasse à la verticale. La cheville retourne en position neutre. Le piston de cheville redescend. L'huile passe de la chambre inférieure à la chambre supérieure par la régulation de dorsiflexion, c'est-à-dire par l'électrovanne (30), jusqu'à obstruction des orifices (11) par le tiroir (9). La cheville est alors en position neutre.

En référence à la figure 5C, lors de la propulsion en avant, c'est-à-dire de 30 à 48% du cycle de marche (fin appui unipodal) représenté sur la figure 3, une fois que le tibia passe la verticale, la cheville est bloquée en dorsiflexion ce qui permet de déformer la lame de pied pour emmagasiner de l'énergie. Lorsque l'on arrive sur l'avant pied, l'instruction est donnée par le microprocesseur pour modifier la résistance de flexion du genou, en pilotant l'électrovanne (25), à un niveau faible. La vanne tout ou rien (13) dite de synergie est désactivée en même temps qu'on autorise la flexion du genou ce qui permet de rigidifier la cheville.

En référence à la figure 5D, lors de l'initiation de la flexion du genou, c'est-à-dire de 48 à 60% du cycle de marche (second appui bipodal) représenté sur la figure 3, l'initiation de la flexion du genou est alors facile. La cheville est toujours bloquée afin de permettre la restitution d'énergie du pied. L'huile passe de la chambre inférieure du vérin (6) de genou à la chambre supérieure en étant régulée par la régulation de flexion, c'est-à-dire par l'électrovanne (25).

En référence à la figure 5E, lors de la flexion en phase oscillante, c'est-à-dire de 60 à 80% du cycle de marche représenté sur la figure 3, la flexion du genou entre 30° et 60° entraine une dorsiflexion de la cheville de manière automatique avec un rapport de 1/3 soit 10° de dorsiflexion de cheville. Le rapport est de 1/3 préférentiellement mais peut être compris entre 1/4 et 2/5. La régulation de flexion, c'est-à-dire l'électrovanne (25) est pilotée électriquement afin d'être asservie à la vitesse de marche ou au poids de la prothèse (inertie de la partie en dessous du genou).

En référence à la figure 5F, lors du retour en extension du genou en phase oscillante, c'est-à-dire de 80 à 100% du cycle de marche représenté sur la figure 3, une fois que la flexion maximale est atteinte, le genou va revenir en extension. A partir de l'information de flexion maximale, l'instruction est donnée par le microprocesseur de bloquer la flexion du genou au cas où la personne trébuche. La remontée du piston de genou doit être rapide. Ce mouvement d'extension est régulé par la vanne d'extension, c'est-à-dire l'électrovanne (20). Celle-ci laisse le fluide passer facilement jusqu'à quasiment 5° de flexion. La vanne va alors progressivement se fermer pour amortir l'impact terminal, c'est-à-dire le retour en extension complète du genou. La vanne tout ou rien (13) dite de synergie peut être soit fermée pour conserver la dorsiflexion pendant le retour en extension du genou soit ouverte à un moment judicieux du retour en extension pour que l'avant du pied soit le plus loin possible du sol.

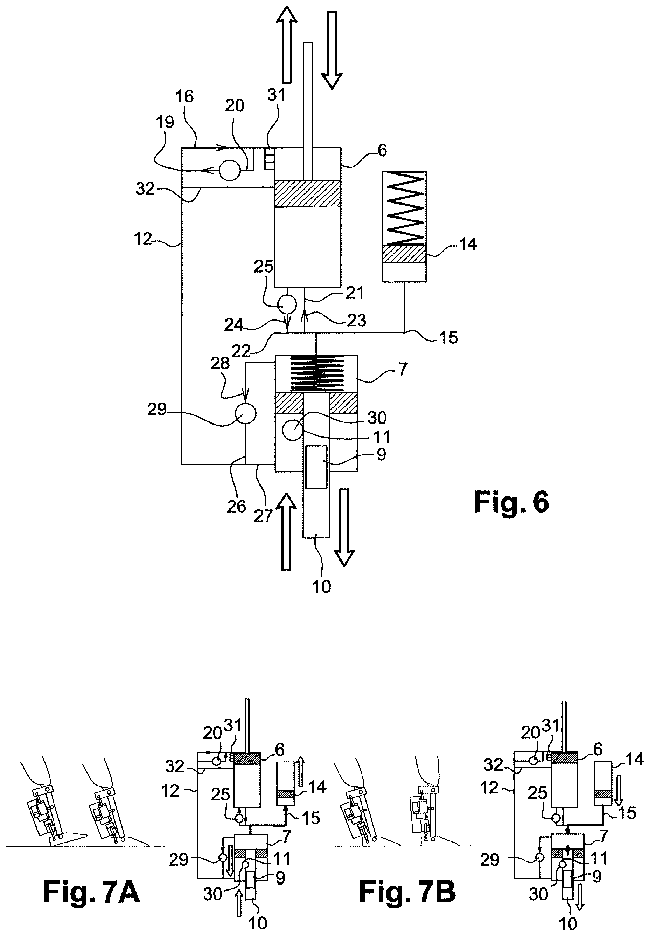

Selon une variante d'exécution de la prothèse suivant l'invention, en référence à la figure 6, la prothèse comporte de la même manière que précédemment un premier vérin hydraulique (6) double effet dont les extrémités sont respectivement solidaires du segment fémoral (1) et du segment tibial (2), et un second vérin hydraulique (7) double effet dont les extrémités sont respectivement solidaires du segment tibial (2) et du pied (4). La chambre supérieure du premier vérin hydraulique (6) est connectée à la chambre inférieure du second vérin hydraulique (7) au moyen d'une conduite (12).

Cette prothèse diffère de la précédente par le fait qu'elle ne comporte pas de vanne tout ou rien (13) pilotée par le microprocesseur. Cette vanne tout ou rien (13) et la conduite (31) connectant l'une des voies de la vanne tout ou rien (13) à la conduite (15) sont substituées par une ou plusieurs conduites (31) connectant la chambre du premier vérin hydraulique (6) à la chambre du second vérin hydraulique (7), via l'électrovanne (20) et une conduite (32) connectant la chambre du premier vérin hydraulique (6) à la chambre du second vérin hydraulique (7), lesdites conduites (31,32) étant connectées latéralement à la chambre du premier vérin hydraulique (6) et la conduite (32) débouchant dans la chambre supérieure du premier vérin hydraulique (6) en dessous de la ou des conduites (31) qui débouchent dans la partie supérieure de la chambre supérieure du premier vérin hydraulique (6), de telle sorte que la conduite (32) est obstruée entièrement (pas de passage par les conduits 31 et 32) ou partiellement (passage possible par un des conduits 31) lorsque le genou est en extension complète afin que la flexion du genou autorise une dorsiflexion de cheville proportionnelle au mouvement du genou au cours de la phase d'appui et respectivement que la flexion du genou entraine une dorsiflexion de cheville au cours de la phase oscillante. Ainsi, le passage d'huile liant le vérin (7) de cheville au vérin (6) de genou arrive en haut du vérin (6) de genou et perpendiculairement au piston de genou de telle sorte que la mise sous pression de la chambre inférieure du vérin (7) de cheville n'entraine pas de mouvement du genou lorsque celui-ci est en extension complète.

On expliquera maintenant le fonctionnement de cette variante d'exécution de la prothèse suivant l'invention en référence aux figures 7A à 7F.

En référence à la figure 7A, lors de la plantiflexion, c'est-à-dire de de 0 à 12% du cycle de marche représenté sur la figure 3, l'appui sur le talon provoque un déplacement du piston de cheville vers le haut. L'huile de la chambre supérieure du vérin (7) de cheville passe dans la chambre inférieure via la régulation de plantiflexion, c'est-à-dire via l'électrovanne (29). L'excédent d'huile est envoyé vers l'accumulateur.

L'huile venant de la chambre basse du pied arrive perpendiculairement au piston de genou. L'augmentation de pression n'entraine donc pas de mouvement du genou. Par contre si la personne exerce un couple de flexion sur le genou, ce mouvement est possible (stanceflex) et est régulé par la régulation de flexion de genou, c'est-à-dire par l'électrovanne (25) (résistance importante).

En référence à la figure 7B, lors du retour du tibia à la verticale, c'est-à-dire de 12 à 30% du cycle de marche représenté sur la figure 3, une fois le pied à plat, le tibia repasse à la verticale. La cheville retourne en position neutre. Le piston de cheville redescend. L'huile passe de la chambre basse à la chambre haute par la régulation de dorsiflexion, c'est-à-dire par l'électrovanne (30) (jusqu'à obstruction des orifices par le tiroir (9). La cheville est alors en position neutre.

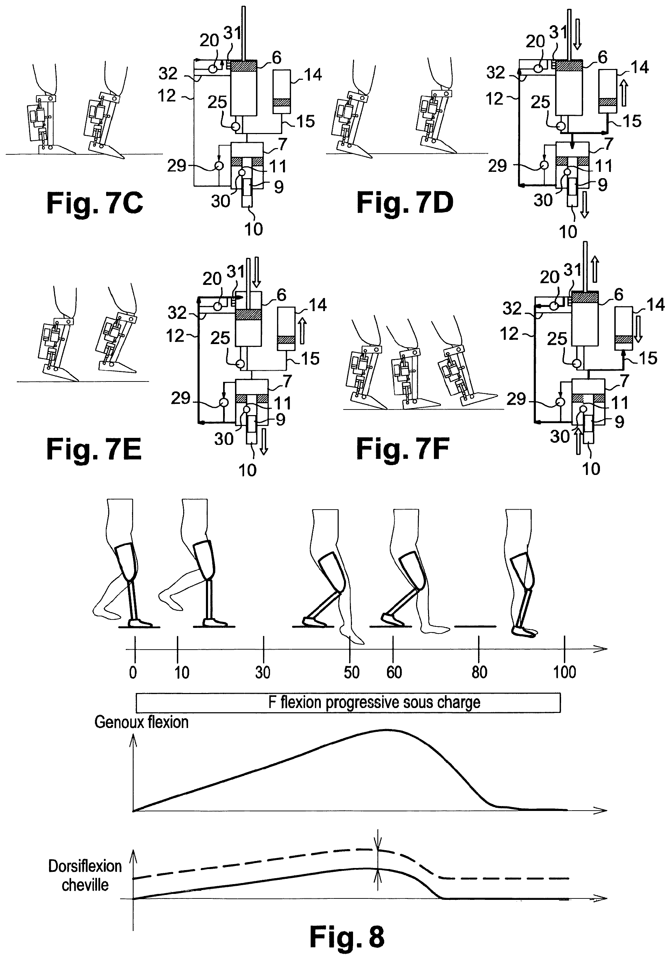

En référence à la figure 7C, lors de la propulsion en avant, c'est-à-dire de 30 à 48% du cycle de marche (fin appui unipodal) représenté sur la figure 3, une fois que le tibia passe la verticale, la cheville est bloquée en dorsiflexion (par la position en extension du genou) ce qui permet de déformer la lame de pied pour emmagasiner de l'énergie. Le retour en extension complète du genou empêche le passage de l'huile venant de la chambre inférieure du vérin (7) de cheville.

En référence à la figure 7D, lors de l'initiation de la flexion du genou, c'est-à-dire de 48 à 60% du cycle de marche (second appui bipodal) représenté sur la figure 3, l'initiation de la flexion du genou est alors facile. L'huile passe de la chambre inférieure du vérin (6) de genou à la chambre supérieure en étant régulée par la régulation de flexion, c'est-à-dire par l'électrovanne (25) (résistance faible). La flexion du genou provoque immédiatement une dorsiflexion de la cheville.

En référence à la figure 7E, lors de la flexion en phase oscillante, c'est-à-dire de 60 à 80% du cycle de marche représenté sur la figure 3, la flexion du genou entre 30° et 60° entraine une dorsiflexion de la cheville de manière automatique avec un rapport de 1/3 soit 10° de dorsiflexion de cheville. Le rapport est de 1/3 préférentiellement mais peut être compris entre 1/4 et 2/5. La régulation de flexion, c'est-à-dire l'électrovanne (25) est pilotée électriquement afin d'être asservie à la vitesse de marche ou au poids de la prothèse (inertie de la partie en dessous du genou).

En référence à la figure 7F, lors du retour en extension du genou en phase oscillante, c'est-à-dire de 80 à 100% du cycle de marche représenté sur la figure 3, une fois que la flexion maximale est atteinte, le genou va revenir en extension. A partir de l'information de flexion maximale, l'instruction est donnée par le microprocesseur de bloquer la flexion du genou au cas où la personne trébuche. La remontée du piston de genou doit être rapide. Ce mouvement d'extension est régulé par la vanne d'extension. Celle-ci laisse le fluide passer facilement jusqu'à quasiment 5° de flexion. La vanne va alors progressivement se fermer pour amortir impact terminal, c'est-à-dire le retour en extension complète du genou.

En référence à la figure 8, en couplant le genou et la cheville, la flexion du genou lors de la descente d'escalier va automatiquement autoriser une dorsiflexion progressive de la cheville. Ceci permet à la personne amputée de placer normalement son pied à plat sur la marche et de ne rouler sur les orteils qu'en fin d'appui. Le couplage « idéal » entre le genou et la cheville est compris entre ¼ et 2/5 et de préférence de l'ordre de 1/3. C'est-à-dire que 60° de flexion du genou entraineront 20° de dorsiflexion de la cheville. Ce couplage peut être plus faible (1/4) pour s'adapter aux situations. Dans ce cas, ou pour s'adapter à des escaliers très raides, la butée de dorsiflexion peut être déplacée pour augmenter la dorsiflexion de la cheville (adaptation représentée en traits pointillés). Le mouvement des deux articulations est contrôlé par la résistance à la flexion du genou, c'est-à-dire par l'électrovanne (25).

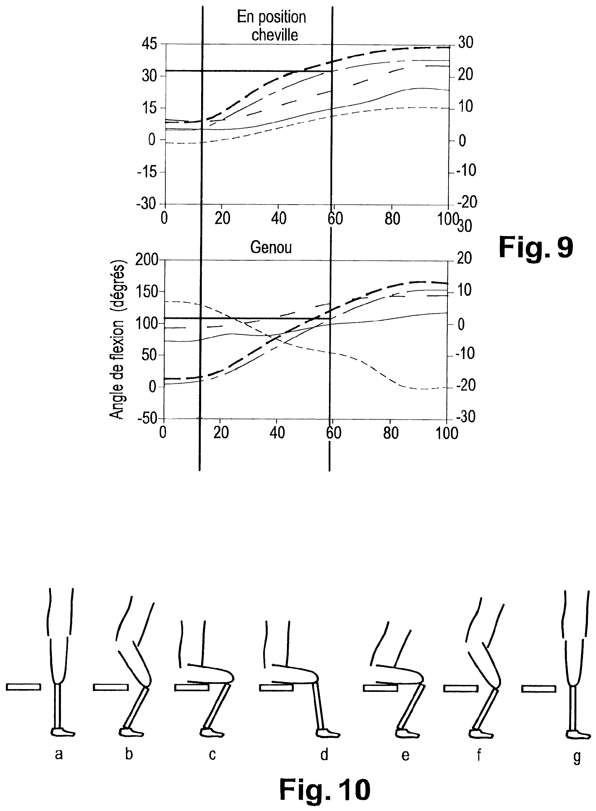

En référence à la figure 9, qui est adaptée de la publication « Hip, knee, and ankle kinematics of high range of motion activities of daily living. Hemmerich A, Brown H, Smith S, Marthandam SS, Wyss UP. J Orthop Res. 2006 Apr;24(4):770-81 », le couplage entre les angles du genou et de la cheville en phase d'appui est particulièrement utile lors des mouvements de triple flexion comme le « squatting ». Cela correspond notamment au mouvement pour ramasser des objets sur le sol ou se mettre en position accroupie. Le contrôle de la flexion du genou permet une mobilité importante et contrôlée du genou et de la cheville qui augmentent la stabilité à la fois statique et dynamique. Ce couplage permet de rendre symétrique la flexion des membres inférieurs et de s'accroupir en conservant le dos droit. Les compensations sont limitées. La dorsiflexion de la cheville est proportionnelle à l'angle du genou, l'amplitude de dorsiflexion est de l'ordre du 1/3 de la flexion du genou soit égale à 33° pour un mouvement de flexion de genou de 100° (de debout à accroupi). Le rapport est préférentiellement de 1/3 mais peut être compris entre 1/4 et 2/5. Ce mouvement est facilement contrôlé par la personne appareillée car le genou et la cheville sont couplés. La personne ne contrôle pas le genou et la cheville séparément mais le mouvement combiné des deux articulations à partir de la flexion extension de sa hanche du membre résiduel.

EP 2 877 130 B1

En référence à la figure 10, lors du passage debout à assis, la flexion du genou est contrôlée par la résistance à la flexion, la flexion du genou induit une dorsiflexion de la cheville, qui permet de maintenir le centre de gravité de la personne en avant de la cheville et en arrière du genou (b). Cela permet une transition stable et sécurisée en position assise (c). Une fois en position assise, seul le ressort de l'accumulateur (14) et la résistance à la dorsiflexion, via l'électrovanne (29) s'opposent à une mise en plantiflexion de la cheville. Ce mouvement est donc rendu possible, ce qui permet une station assise confortable (d). Pour se relever, il suffit de retirer le pied en arrière (avec la main, ou avec l'autre jambe), la cheville est en dorsiflexion proportionnellement à l'angle du genou (e), le passage en position debout se fait par une extension coordonnée du membre inférieur. Ce mouvement est produit par un couple d'extension de hanche qui est d'autant plus faible que le centre de gravité est proche de l'articulation de hanche (e à g). Si le lever de chaise est détecté, il semble utile de régler la résistance à la flexion du genou, c'est-à-dire l'électrovanne (25), au maximum afin d'éviter tout basculement en arrière de la personne.

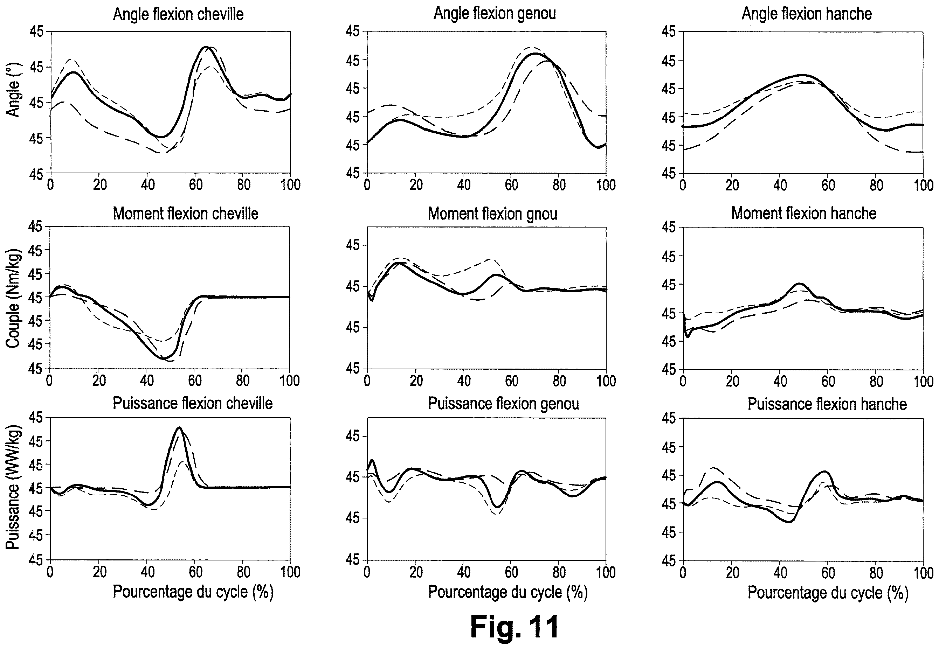

En référence à la figure 11, en descente de pente, l'amplitude de plantiflexion de cheville est maximale. Entre 0 et 12%, l'amplitude de plantiflexion est augmentée, ceci est réalisé par la résistance à la plantiflexion qui peut être ajustée lorsqu'une pente est détectée. L'amplitude nécessaire pour mettre le pied à plat sur le sol est augmentée en descente de pente. Entre 12 et 48% on observe une flexion du genou sous charge contrôlée par la résistance à la flexion, i.e. par l'électrovanne (25). La résistance à la flexion du genou, i.e. l'électrovanne (25), au cours de la phase d'appui peut être adaptée à la valeur de la pente afin d'augmenter la résistance lors des pentes fortes et de la diminuer lors de pentes faibles. A la fin de l'appui unipodal (vers 48%), l'instruction doit être donnée au microcontrôleur de passer en phase oscillante. On peut constater qu'à la différence de la marche à plat, le genou n'est pas en extension complète et que le moment à la cheville est plus faible. Ceci met en évidence que lorsqu'une pente descendante est détectée, il faut modifier les conditions de transition du système entre la phase d'appui et la phase oscillante.

En descente de pente il peut être intéressant de modifier la butée de dorsiflexion selon l'angle de la pente afin de permettre une grande stabilité en cas d'arrêt sur cette pente en adaptant la position du tiroir (9). La position du tiroir (9) ne détermine que la dorsiflexion de la cheville si le genou est en extension Ainsi, la flexion du genou en phase d'appui va autoriser une dorsiflexion automatique de la cheville reproduisant l'adaptation des sujets sains (c'est à dire : augmentation de la dorsiflexion en fin de phase d'appui).

En cas de montée de pente, la butée de dorsiflexion est augmentée en fonction de la pente en adaptant la position du tiroir (9). Ceci permet une dorsiflexion importante dès le début de l'appui unipodal. La résistance à la flexion du genou, via l'électrovanne (25) est portée à son maximum et doit permettre une mise en charge genou fléchi. On peut constater que chez le sujet sain, seule la hanche travaille au début d'appui (puissance positive). Ce travail de la hanche, permet de ramener le genou en extension complète sans puissance interne positive du genou. En cas d'arrêt, la butée de dorsiflexion de la cheville sera adaptée à l'angle de la pente.

La position du tiroir (9) est modifiée par le moteur non représenté sur les figures. Le moteur ne bouge pas la cheville mais déplace le tiroir (9) qui définit la butée de dorsiflexion lorsque le genou est en extension. Le moteur n'est donc utilisé que lors des changements de pente (ou lorsque l'utilisateur change de chaussure) ce qui est économique en énergie.

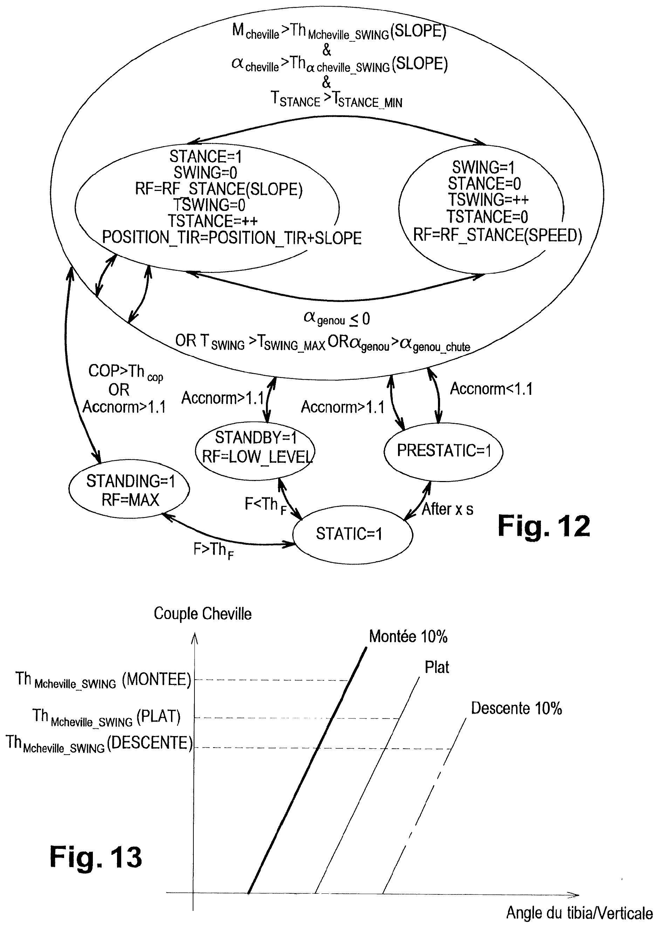

On décrira maintenant le principe de fonctionnement de l'unité de commande de la prothèse suivant l'invention en référence à la figure 12.

Les capteurs cinématiques et inertiels utilisés sont de préférence un capteur d'angle du genou, un capteur d'angle de la cheville et un accéléromètre bi axial ou tri axial combiné à un gyroscope dans le plan sagittal permettant de mesurer l'angle du tibia par rapport à la verticale. A partir de ces trois capteurs, on peut déduire l'angle du fémur et l'angle du pied par rapport au sol dans le plan sagittal.

Les capteurs d'efforts utilisés sont, de préférence, l'effort dans l'axe du tibia, le moment sagittal de la cheville et le moment sagittal en un autre point de la prothèse. Ces efforts et moments peuvent être avantageusement déterminés par une ou plusieurs mesures simples. Par exemple, le moment à la cheville peut être déterminé par la mesure de l'effort de traction/compression le long de la bielle (8) liant l'unité hydraulique au pied (figure 1).

A partir de ces 6 capteurs, on peut déduire la composante antéropostérieure et le centre de pression de l'effort exercé par le sol sur la prothèse. Dans le plan sagittal, on connait donc tous les angles et le torseur des actions exercées par le sol sur la prothèse. D'autres capteurs peuvent être ajoutés pour permettre de la redondance et la mesure dans les deux autres plans de l'espace.

Les variables de l'algorithme sont les suivantes :

| Variable | Description |
|---|---|
| SLOPE | Estimation de la pente sur laquelle l'utilisateur marche |
| SPEED | Estimation de la vitesse sur laquelle l'utilisateur marche |
| $\alpha_{GENOU}$ | Valeur courante de l'angle du genou |

(suite)

| Variable | Description |
|---|---|
| $Th_{\alpha\, GENOU\_SWING(SLOPE)}$ | Seuil de retour en extension du genou permettant le passage en phase oscillante. Ce seuil dépend de la pente (SLOPE) |
| $M_{CHEVILLE}$ | Valeur courante du moment à la cheville |
| $Th_{MCHEVILLE\_SWING(SLOPE)}$ | Seuil de moment à la cheville permettant le passage en phase oscillante. Ce seuil dépend de la pente (SLOPE) |
| $T_{STANCE}$ | Durée de la phase d'appui |
| $T_{STANCE\_MIN}$ | Durée minimale de la phase d'appui avant passage en phase oscillante |
| RF | Valeur courante de la résistance à la flexion du genou (électrovanne 25) |
| RF_STANCE(SLOPE) | Résistance à la flexion du genou en phase d'appui. Cette valeur dépend de la pente (SLOPE) |
| RF_SWING(SPEED) | Résistance à la flexion du genou en phase oscillante. Cette valeur dépend de la vitesse de marche (SPEED) |
| POSITION_TIR | Position du tiroir de butée de dorsiflexion |
| $T_{SWING}$ | Durée de la phase oscillante |
| $T_{SWING\_MAX}$ | Durée maxi de la phase oscillante |
| $\dot{\alpha}_{genou}$ | Valeur courante de la Vitesse de flexion du genou |
| $\dot{\alpha}_{genou\_chute}(SPEED)$ | Seuil de vitesse de flexion de genou au-dessus de laquelle on considère que la personne est en train de tomber. Cette variable dépend de la vitesse de marche (SPEED). |
| Accnorm | Norme des trois composantes de l'accéléromètre placé sur le tibia |
| F | Effort dans l'axe du tibia |
| $Th_F$ | Seuil d'effort sur la prothèse pour lequel on considère que la prothèse est en charge |
| $\dot{COP}$ | Variation du centre de pression. Le centre de pression peut être estimé de manière grossière par le ratio entre le moment à la cheville et l'effort dans l'axe du tibia. |

On observera que, dans un mode de réalisation, l'ensemble des capteurs n'est pas nécessaire. Les capteurs de mesure de l'angle du genou, le moment à la cheville, et l'angle du tibia par rapport à la verticale et l'effort dans l'axe du tibia sont suffisants.

En référence à la figure 12, par défaut, le système est dans l'état **STANCE**, la résistance à la flexion de genou (RF) est à une valeur élevée permettant de freiner le genou sous charge (RF_STANCE(SLOPE)). La valeur de la résistance à la flexion du genou sous charge peut être modifiée en fonction de la pente (SLOPE) sur laquelle la personne évolue. De même, la position de la butée de dorsiflexion POSITION_TIR est ajustée en fonction de la pente (SLOPE). La fin de la phase d'appui est détectée lorsque la personne est en appui sur l'avant de son pied prothétique. Ceci se traduit par un moment de dorsiflexion important au niveau de la cheville $M_{CHEVILLE}$. Un seuil $Th_{MCHEVILLE\_SWING(SLOPE)}$ est réglé par l'orthoprothésiste en fonction du poids et du style de marche de la personne appareillée. Ce seuil peut être modifié par le contrôleur en fonction de la pente (SLOPE). La fin de l'appui se traduit également par un genou proche de l'extension complète $\alpha_{GENOU} < Th_{\alpha\, GENOU\_SWING(SLOPE)}$. Ce seuil d'extension du genou peut également être modifié par le contrôleur en fonction de la pente. Afin d'éviter un passage en phase oscillante en tout début d'appui, un temps minimal de phase d'appui est requis ($T_{STANCE} > T_{STANCE\_MIN}$). Ces trois conditions permettent le passage à l'état **SWING**.

Lors de l'état **SWING**, la résistance à la flexion du genou RF est à la valeur RF_SWING(SPEED). Ce niveau de résistance à la flexion permet une flexion du genou en phase oscillante. Cette valeur est modifiée en fonction de la vitesse de marche de la personne appareillée.

Le système revient à l'état **STANCE** si une des 4 conditions suivantes se produit :

- $\dot{\alpha}_{genou} < 0,$ c'est-à-dire que le genou a atteint sa flexion maximale et revient en extension. La valeur de RF n'intervient plus dans le mouvement du genou. RF est donc remis à RF_STANCE pour préparer la phase d'appui suivante

- $T_{SWING} > T_{SWING\_MAX}$ la durée de l'état SWING est trop longue.

- $\dot{\alpha}_{genou} > \dot{\alpha}_{genou\_chute},$ la vitesse de flexion du genou est supérieure à une vitesse de flexion attendue en fonction de la vitesse de marche de la personne, il y a risque de chute.

- $F > Th_F$ : Un effort est détecté dans l'axe de la prothèse.

Alternativement, la condition $\dot{\alpha}_{genou} < 0$ pourra être substituée par la condition $\dot{\alpha}_{tibia} < 0,$ en utilisant un gyroscope placé sur le tibia ou sur l'unité hydraulique par exemple.

Si la norme des composantes de l'accéléromètre est inférieure à 1.1g, ou tout autre seuil prédéterminé, par des essais par exemple, cela signifie que le tibia ne bouge pas. Par extension, en contrôlant l'angle du genou et de la cheville, on peut vérifier que l'ensemble du membre prothétique est immobile. On est alors dans un état **PRESTATIC**. Si cet état se maintient pendant un certain temps, on passe à l'état **STATIC**. S'il n'y a pas d'effort sur la prothèse (F), on passe directement à l'état **STANDBY** et on peut baisser la résistance à la flexion au maximum. A l'inverse s'il y a un effort sur la prothèse, on passe à l'état **STANDING**, la résistance à la flexion est portée à un niveau maximal bloquant la flexion du genou.

Dès que la norme des composantes de l'accéléromètre est supérieur à 1.1g ou que le centre de pression (cop ~ $M_{CHEVILLE}/F$) varie, on repasse à l'état **STANCE**.

Plus généralement, on peut repasser à l'état **STANCE**, dès que l'on détecte un mouvement, ou une modification de l'état de chargement de la prothèse.

La vitesse de marche peut être estimée à partir d'un très grand nombre de variables mesurée par le système (temps de phase d'appui, vitesse max de flexion du genou, temps de phase oscillante, vitesse angulaire du tibia,etc...).

La figure 13 est une représentation graphique sans échelle du couple de la cheville en fonction de l'angle du tibia par rapport à la verticale lors de la marche sur le plat, en montée et en descente de pente entre 30% et 50% du cycle de marche. La montée de pente entraine un décalage de cette courbe sur la gauche, à l'inverse une descente de pente entraine un décalage de cette courbe sur la droite. La valeur de ce décalage est liée à la pente sur laquelle l'utilisateur marche et permet donc de déterminer SLOPE.

En montée de pente, le seuil $Th_{MCHEVILLE\_SWING(PLAT)}$ est donc atteint plus tôt dans le cycle de marche. Ce seuil $Th_{MCHEVILLE\_SWING(MONTEE)}$ peut donc être augmenté afin de retarder le passage en phase oscillante.

A l'inverse, en descente de pente, le seuil $Th_{MCHEVILLE\_SWING(PLAT)}$ est atteint plus tard dans le cycle de marche. Le seuil $Th_{MCHEVILLE\_SWING(DESCENTE)}$ doit être donc diminué dans le cas d'une descente de pente, afin de passer plus facilement en phase oscillante.

Pour obtenir l'angle du tibia par rapport à la verticale, on peut utiliser un accéléromètre et/ou un gyroscope sur le tibia ou sur l'unité hydraulique.

Enfin, il est bien évident que les exemples que l'on vient de donner ne sont que des illustrations particulières en aucun cas limitatives quant aux domaines d'application de l'invention.

## Revendications

1. Prothèse fémorale genou-cheville destinée à des personnes amputées du membre inférieur comportant un segment fémoral (1) apte à être fixé à une connexion fémorale, et un segment tibial (2) articulé au segment fémoral (1) autour d'une articulation (3) reproduisant les mouvements du genou, ledit segment tibial (2) étant articulé à un pied (4) autour d'une articulation (5) reproduisant les mouvements de la cheville, un premier vérin hydraulique (6) dont les extrémités sont respectivement solidaires du segment fémoral et du segment tibial, et un second vérin hydraulique (7) dont les extrémités sont respectivement solidaires du segment tibial (2) et du pied (4) par l'intermédiaire d'une bielle (8), la chambre supérieure du premier vérin hydraulique (6) est connectée à la chambre inférieure du second vérin hydraulique (7) au moyen d'au moins une conduite (12,31,32) connectée latéralement à la chambre supérieure du premier vérin hydraulique, *caractérisée* **en ce que** la prothèse comporte une unité de commande configurée pour piloter ledit premier vérin hydraulique (6) et/ou ledit second vérin hydraulique (7) en fonction de la phase de la marche telle que la phase d'appui ou la phase oscillante et/ou des situations de la vie courant telles que les escaliers, les pentes, ou une station debout ou analogue, de telle manière que la flexion du genou autorise une dorsiflexion de cheville proportionnelle au mouvement du genou au cours de la phase d'appui et de telle manière que la flexion du genou entraîne une dorsiflexion de cheville au cours de la phase oscillante.

2. Prothèse fémorale genou-cheville suivant la revendication 1 *caractérisée* **en ce que** les déplacements des vérins (6,7) sont régulés par une ou plusieurs vanne(s) (20,25,29,30)

3. Prothèse fémorale genou cheville suivant la revendication 2 *caractérisée* **en ce que** la ou lesdites vanne(s) (20,25,29,30) sont contrôlées par l'unité de commande en fonction de la phase du cycle de marche et de la situation dans laquelle la personne se trouve.

4. Prothèse fémorale genou-cheville suivant l'une quelconque des revendications 1 à 3 *caractérisée* **en ce que** l'unité de commande est configurée pour déterminer la phase de marche et/ou la situation à partir de données issues de capteurs placés sur l'articulation de genou et/ou l'articulation de cheville et/ou les segments adjacents à ces articulations.

5. Prothèse fémorale genou-cheville suivant la revendication 4 *caractérisée* **en ce que** chaque capteur consiste en un capteur cinétique, cinématique ou inertiel.

6. Prothèse fémorale genou-cheville suivant l'une quelconque des revendications 1 à *5 caractérisée* **en ce que** l'unité de commande est configurée pour piloter les vannes de régulations (20,25,29,30) des vérins (6,7) de manière à faire varier les résistances à la flexion/extension de l'articulation de genou et/ou de l'articulation de cheville.

7. Prothèse fémorale genou-cheville suivant l'une quelconque des revendications 1 à *6 caractérisée* **en ce que** le rapport entre la flexion du genou et la dorsiflexion de cheville est compris entre 1/4 et 2/5.

8. Prothèse fémorale genou-cheville suivant la revendication 7 *caractérisée* **en ce que** le rapport entre la flexion du genou et la dorsiflexion de cheville est sensiblement égal à 1/3.

9. Prothèse fémorale genou-cheville suivant l'une quelconque des revendications 1 à 8 *caractérisée* **en ce que** l'unité de commande comporte au moins un microprocesseur.

10. Prothèse fémorale genou-cheville suivant l'une quelconque des revendications 1 à 9 *caractérisée* **en ce que** l'unité de commande composite
un algorithme déterminant le moment de l'articulation de genou ou de cheville
en fonction d'un angle d'un segment adjacent à l'articulation par rapport à un référentiel et/ou de la vitesse angulaire du segment adjacent à l'articulation dans un référentiel déterminé et un algorithme déterminant une instruction de pilotage des vérins 6,7
en fonction du rapport entre le moment de force de l'articulation et un angle d'un segment adjacent à l'articulation par rapport à un référentiel.

**Patentansprüche**

1. Oberschenkelprothese mit Knie- und Sprunggelenk für Personen mit Beinamputation, wobei diese einen Oberschenkelabschnitt (1) aufweist, der dazu geeignet ist, an einer Oberschenkelverbindung befestigt zu werden, sowie einen Schienbeinabschnitt (2), der über ein Gelenk (3), welches die Kniebewegung nachbildet, am Oberschenkelabschnitt (1) angelenkt ist, wobei der Schienbeinabschnitt (2) über ein Gelenk (5), welches die Bewegung des Sprunggelenks nachbildet, an einem Fuß (4) angelenkt ist, sowie einen ersten hydraulischen Stellzylinder (6), dessen Enden mit dem Oberschenkelabschnitt beziehungsweise mit dem Schienbeinabschnitt fest verbunden sind, und einen zweiten hydraulischen Stellzylinder (7), dessen Enden mit dem Schienbeinabschnitt (2) beziehungsweise, über eine Schubstange (8), mit dem Fuß (4) fest verbunden sind, wobei die obere Kammer des ersten hydraulischen Stellzylinders (6) mittels mindestens einer Leitung (12, 31, 32), welche seitlich an die obere Kammer des ersten hydraulischen Stellzylinders angeschlossen ist, mit der unteren Kammer des zweiten hydraulischen Stellzylinders (7) verbunden ist,
*dadurch gekennzeichnet,* **dass** die Prothese eine Steuereinheit aufweist, die dafür ausgelegt ist, den ersten hydraulischen Stellzylinder (6) und/oder den zweiten hydraulischen Stellzylinder (7) in Abhängigkeit von der Gangphase, wie etwa der Standphase oder der Schwungphase, und/oder von Alltagssituationen wie Treppen, Gefällestrecken oder einem aufrechten Stehen oder Ähnlichem derart zu steuern, dass die Beugung des Kniegelenks im Laufe der Standphase eine Dorsalflexion des Sprunggelenks gestattet, die proportional zur Bewegung des Kniegelenks ist, und dass die Beugung des Kniegelenks im Laufe der Schwungphase eine Dorsalflexion des Sprunggelenks bewirkt.

2. Oberschenkelprothese mit Knie- und Sprunggelenk gemäß Anspruch 1, *dadurch gekennzeichnet,* **dass** die Bewegungen der Stellzylinder (6, 7) von einem oder mehreren Ventil(en) (20, 25, 29, 30) reguliert werden.

3. Oberschenkelprothese mit Knie- und Sprunggelenk gemäß Anspruch 2, *dadurch gekennzeichnet,* **dass** das oder die Ventil(e) (20, 25, 29, 30) von der Steuereinheit in Abhängigkeit von der Phase des Gangzyklus und der Situation, in welcher sich die Person befindet, gesteuert wird/werden.

4. Oberschenkelprothese mit Knie- und Sprunggelenk gemäß einem beliebigen der Ansprüche 1 bis 3, *dadurch gekennzeichnet,* **dass** die Steuereinheit dafür ausgelegt ist, die Gangphase und/oder die Situation ausgehend von Daten zu bestimmen, die von Sensoren stammen, welche am Kniegelenk und/oder am Sprunggelenk und/oder an den Abschnitten, die an diese Gelenke angrenzen, angebracht sind.

5. Oberschenkelprothese mit Knie- und Sprunggelenk gemäß Anspruch 4, *dadurch gekennzeichnet,* **dass** jeder der Sensoren aus einem kinetischen Sensor, einem kinematischen Sensor oder einem Trägheitssensor besteht.

6. Oberschenkelprothese mit Knie- und Sprunggelenk gemäß einem beliebigen der Ansprüche 1 bis 5, *dadurch gekennzeichnet,* **dass** die Steuereinheit dafür ausgelegt ist, die Regulierungsventile (20, 25, 29, 30) der Stellzylinder (6, 7) derart zu steuern, dass der jeweilige Widerstand gegen eine Beugung/Streckung des Kniegelenks und/oder des Sprunggelenks verändert wird.

7. Oberschenkelprothese mit Knie- und Sprunggelenk gemäß einem beliebigen der Ansprüche 1 bis 6, *dadurch gekennzeichnet,* **dass** das Verhältnis zwischen der Beugung des Kniegelenks und der Dorsalflexion des Sprunggelenks zwischen 1/4 und 2/5 beträgt.

8. Oberschenkelprothese mit Knie- und Sprunggelenk gemäß Anspruch 7, *dadurch gekennzeichnet,* **dass** das Verhältnis zwischen der Beugung des Kniegelenks und der Dorsalflexion des Sprunggelenks im Wesentlichen gleich 1/3 ist.

9. Oberschenkelprothese mit Knie- und Sprunggelenk gemäß einem beliebigen der Ansprüche 1 bis 8, *dadurch gekennzeichnet,* **dass** die Steuereinheit mindestens einen Mikroprozessor aufweist.

10. Oberschenkelprothese mit Knie- und Sprunggelenk gemäß einem beliebigen der Ansprüche 1 bis 9, *dadurch gekennzeichnet,* **dass** die Steuereinheit einen Algorithmus aufweist, der das Moment des Kniegelenks oder des Sprunggelenks festlegt, und zwar in Abhängigkeit von einem Winkel eines Abschnitts, der an das Gelenk angrenzt, im Abgleich mit einer Gesamtheit von Bezugswerten, und/oder von der Winkelgeschwindigkeit des Abschnitts, der an das Gelenk angrenzt, innerhalb einer bestimmten Gesamtheit von Bezugswerten, sowie einen Algorithmus, der einen Befehl zur Steuerung der Stellzylinder 6, 7 festlegt, und zwar in Abhängigkeit vom Verhältnis zwischen dem Kraftmoment des Gelenks und einem Winkel eines Abschnitts, der an das Gelenk angrenzt, im Abgleich mit einer Gesamtheit von Bezugswerten.

**Claims**

1. Femoral knee-ankle prosthesis intended for persons who are lower-limb amputees, having a femoral segment (1) suitable to be fixed to a femoral connection, and a tibial segment (2) hinged to the femoral segment (1) via an hinge (3) reproducing the movements of the knee, said tibial segment (2) being hinged to a foot (4) via an hinge (5) reproducing the movements of the ankle, a first hydraulic cylinder (6) the ends of which are integrally joined respectively with the femoral and tibial segments, a second hydraulic cylinder (7) the ends of which are integrally joined respectively with the tibial segment (2) and the foot (4) by means of a connecting rod (8); the upper chamber of the first hydraulic cylinder (6) is connected to the lower chamber of the second hydraulic cylinder (7) via at least one duct (12, 31, 32) connected laterally to the upper chamber of the first hydraulic cylinder, **characterized in that** the prosthesis comprises a control unit configured to control said first hydraulic cylinder (6) and/or said second hydraulic cylinder (7) depending on the phase of the walking, such as the stance phase or the swing phase and/or real-life situations such as stairs, slopes or standing position or the like, in such a manner that the flexion of the knee allows a dorsiflexion of the ankle in proportion to the movement of the knee during the stance phase and that the flexion of the knee results in the dorsiflexion of the ankle during the swing phase.

2. Femoral knee-ankle prosthesis according to claim 1, **characterized in that** the displacements of the cylinders (6,7) are controlled by one or more valves (20, 25, 29, 30).

3. Femoral knee-ankle prosthesis according to claim 2, **characterized in that** said one or more valves (20, 25, 29, 30) are controlled by the control unit depending on the phase of the walking cycle and the situation in which the person is.

4. Femoral knee-ankle prosthesis according to any one of claims 1 to 3, **characterized in that** the control unit is configured to determine the walking phase and/or the situation based on data obtained from the sensors placed on the knee joint and/or ankle joint and/or the segments adjacent to theses joints.

5. Femoral knee-ankle prosthesis according to claim 4, **characterized in that** each sensor consists of a kinetic, kinematic or inertial sensor.

6. Femoral knee-ankle prosthesis according to any one of claims 1 to 5, **characterized in that** the control unit is configured to control the regulating valves (20, 25, 29, 30) of the cylinders (6, 7) in order to vary the flexion/extension resistances of the knee joint and/or ankle joint.

7. Femoral knee-ankle prosthesis according to any one of claims 1 to 6, **characterized in that** the ratio between the flexion of the knee and the dorsiflexion of the ankle is between 1/4 and 2/5.

8. Femoral knee-ankle prosthesis according to claim 7, **characterized in that** the ratio between the flexion of the knee and the dorsiflexion of the ankle is approximately 1/3.

9. Femoral knee-ankle prosthesis according to any one of claims 1 to 8, **characterized in that** the control unit comprises at least one microprocessor.

10. Femoral knee-ankle prosthesis according to any one of claims 1 to 9, **characterized in that** the control unit comprises an algorithm that determines the movement of knee joint or ankle joint depending on an angle of a segment adjacent to the joint relative to a reference and/or the angular speed of the segment adjacent to the joint in a determined reference and an algorithm that determines a control instruction of the cylinders (6, 7) depending on the ratio between the force moment of the joint and an angle of a segment adjacent to the joint relative to a reference.

**Fig. 1**

**Fig. 2**

**Fig. 3**

**Fig. 4**

Fig. 5A

Fig. 5B

Fig. 5C

Fig. 5D

Fig. 5E

Fig. 5F

Fig. 6

Fig. 7A    Fig. 7B

Fig. 7C

Fig. 7D

Fig. 7E

Fig. 7F

F flexion progressive sous charge

Genoux flexion

Dorsiflexion cheville

Fig. 8

En position
cheville

Angle de flexion (dégrés)

Genou

**Fig. 9**

a   b   c   d   e   f   g

**Fig. 10**

Fig. 11

$M_{cheville} > Th_{Mcheville\_SWING}(SLOPE)$
&
$\alpha_{cheville} > Th_{\alpha cheville\_SWING}(SLOPE)$
&
$T_{STANCE} > T_{STANCE\_MIN}$

STANCE=1
SWING=0
RF=RF_STANCE(SLOPE)
TSWING=0
TSTANCE=++
POSITION_TIR=POSITION_TIR+SLOPE

SWING=1
STANCE=0
TSWING=++
TSTANCE=0
RF=RF_STANCE(SPEED)

$\alpha_{genou} \leq 0$
OR $T_{SWING} > T_{SWING\_MAX}$ OR $\alpha_{genou} > \alpha_{genou\_chute}$

COP>Th$_{cop}$
OR
Accnorm>1.1

Accnorm>1.1

STANDBY=1
RF=LOW_LEVEL

Accnorm>1.1

Accnorm<1.1

PRESTATIC=1

STANDING=1
RF=MAX

F<Th$_F$

After x s

STATIC=1

F>Th$_F$

**Fig. 12**

Couple Cheville

Montée 10%

Plat

Th$_{Mcheville\_SWING}$ (MONTEE)

Th$_{Mcheville\_SWING}$ (PLAT)

Th$_{Mcheville\_SWING}$ (DESCENTE)

Descente 10%

**Fig. 13**

Angle du tibia/Verticale

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 20040111163 A
- US 20060122711 A
- WO 9641599 A
- EP 549855 A
- WO 20050283257 A
- WO 2005087144 A
- WO 20050197717 A
- WO 20070050047 A
- WO 2008103917 A
- US 6443993 B
- US 2478721 A

**Littérature non-brevet citée dans la description**

- **HEMMERICH A ; BROWN H ; SMITH S ; MAR-THANDAM SS ; WYSS UP.** *J Orthop Res.,* Avril 2006, vol. 24 (4), 770-81